# EUROPÄISCHE PATENTSCHRIFT

(11) **EP 3 936 069 B1**
(45) Veröffentlichungstag und Bekanntmachung des Hinweises auf die Patenterteilung: **07.01.2026**
(21) Anmeldenummer: 21182114.5
(22) Anmeldetag: 28.06.2021
(51) Int. Cl.: A61B 18/14

(54) **HOCHFREQUENZELEKTRODE ZUR VERWENDUNG IN EINEM CHIRURGISCHEN HANDGERÄT, ELEKTRODENINSTRUMENT UND RESEKTOSKOP**
HIGH-FREQUENCY ELECTRODE FOR USE IN A SURGICAL HAND-HELD DEVICE, ELECTRODE INSTRUMENT AND RESECTOSCOPE
ÉLECTRODE HAUTE FRÉQUENCE DESTINÉE À ÊTRE UTILISÉE DANS UN APPAREIL CHIRURGICAL PORTABLE, INSTRUMENT À ÉLECTRODE ET RÉSECTOSCOPE

(30) Priorität: 07.07.2020 DE 102020117810
(43) Veröffentlichungstag der Anmeldung: 12.01.2022
(73) Patentinhaber: Olympus Winter & Ibe GmbH, 22045 Hamburg (DE)
(72) Erfinder: Carroux, Alexander, 22885 Barsbüttel (DE); Wosnitza, Thomas, 21337 Lüneburg (DE)
(74) Vertreter: Hoener, Matthias

(56) Entgegenhaltungen:
- EP-A1- 1 791 483
- WO-A1-97/07747
- US-A- 5 843 019
- US-A1- 2001 053 908
- US-A1- 2010 331 621
- US-A1- 2017 100 190
- US-A1- 2018 344 382
- US-A1- 2019 298 444
- US-B1- 6 544 260
- US-B1- 6 730 081

## Beschreibung

Die Erfindung betrifft eine Hochfrequenzelektrode zur Verwendung in einem chirurgischen Handgerät gemäß dem Oberbegriff des Anspruchs 1. Des Weiteren betrifft die Erfindung ein Elektrodeninstrument zur Verwendung in einem chirurgischen Handgerät gemäß dem Anspruch 10 sowie ein Resektoskop gemäß dem Anspruch 11 Hochfrequenzelektroden für Handgeräte, insbesondere Resektoskope, der gattungsgemäßen Art werden vor allem in der Urologie bei elektrochirurgischen Arbeiten in der Blase, Prostata und der Urethra verwendet. Gleichermaßen können diese Hochfrequenzelektroden auch für andere chirurgische sowie orthopädische Behandlungen bzw. Eingriffe verwendet werden. In der Regel werden diese Elektroden zur Resektion und Vaporisation sowie Elektrokoagulation von Gewebe, z. B. von Gewebe im unteren Harntrakt, verwendet. Dazu umfassen die Handgeräte bzw. die Resektoskope eine gattungsgemäße Hochfrequenzelektrode bzw. ein Elektrodeninstrument, das innerhalb eines Schaftes des Handgeräts bzw. des Resektoskopes längsverschieb- und rotierbar gelagert ist. Die chirurgische Hochfrequenzelektrode ist an einem distalen Arbeitsende des Elektrodeninstrumentes, z. B. in Form einer Schlinge oder eines Knopfes angeordnet.

Derartige Elektroden können monopolar oder bipolar ausgebildet sein. Je nach Anwendungsfall bzw. Ausführungsform werden die Elektroden dazu über elektrische Leiter mit elektrischer Energie bzw. einer elektrischen Hochfrequenzspannung beaufschlagt. Die Versorgung mit dem hochfrequenten Strom erfolgt über das Handgerät bzw. das Resektoskop durch einen Hochfrequenzgenerator. Für den Fall, dass die Elektrode als monopolare Elektrode ausgebildet ist, wird eine Neutralelektrode an der zu behandelnden Person angeordnet. Alternativ kann die Neutralelektrode auch Bestandteil des Resektoskops sein. Dabei dienen der Schaft, der Transporteur und die Optik als Neutralelektrode eines elektrischen Potentials.

Durch die Beaufschlagung der Elektrode mit einer üblichen Hochfrequenzspannung bildet sich um die Elektrode bzw. um einige Teile der Elektrode ein Plasma elektrisch geladener Teilchen aus. Das Plasma ist direkt an der Elektrode lokalisiert und weist eine hohe Temperatur bzw. Energiedichte auf, weswegen es sich besonders gut für die oben genannten Einsatzzwecke eignet. Insbesondere für die Vaporisation und die Elektrokoagulation von Gewebe ist es wesentlich, dass das Plasma an einer bestimmten Fläche der Elektrode erzeugt wird, sodass die behandelnde Person die Elektrode zielgenau einsetzen kann.

Bei bekannten Hochfrequenzelektroden bildet sich zum Teil ein Plasma aus, das eher zu den elektrischen Leitern der Elektrode hin orientiert ist. Dadurch lässt sich die Elektrode nicht gezielt einsetzen und die Plasmadichte an der Elektrode selbst verringert sich. Idealerweise bildet sich das Plasma an einer unteren bzw. einem mittleren Bereich der Elektrode aus, an dem die Elektrode in Kontakt mit dem Gewebe steht. Die Ausbildung des Plasmas in einem oberen Bereich der Elektrode, wo sich die elektrischen Leiter befinden, erweist sich für den chirurgischen Einsatz als ungünstig.

Für die oben genannten Behandlungen ist es vorteilhaft, wenn die Elektrode eine möglichst große aktive Fläche aufweist. Die Größe der Fläche ist allerdings zum einen eingeschränkt durch die Dimension des Einsatzbereichs, zum anderen ist für das Zünden des Plasmas für große Elektroden eine höhere Energie notwendig. Eine erhöhte Hitze wirkt sich allerdings nachteilig auf den zu behandelnden Bereich im Körper aus. Außerdem wird durch eine größere Elektrode die Zündgeschwindigkeit reduziert. Relevanter Stand der Technik findet sich in US 2018/344382 A1, WO 97/07747 A1, US 6 730 081 B1, US 6 544 260 B1, US 2017/100190 A1.

Der Erfindung liegt die Aufgabe zugrunde, eine Hochfrequenzelektrode, ein Elektrodeninstrument sowie ein Resektoskop zu schaffen, bei dem sich das Plasma besser an der Elektrode lokalisieren lässt, wodurch die in Gewebekontakt befindliche Fläche maximiert wird und zugleich die Energie zum Zünden des Plasmas reduziert ist.

Eine Lösung dieser Aufgabe wird durch die Merkmale des Anspruchs 1 beschrieben. Demnach ist es vorgesehen, dass die Hochfrequenzelektrode zur Verwendung in einem chirurgischen Handgerät, insbesondere einem Resektoskop, die Form eines Toroids aufweist. Diese toroid- oder donutartige Elektrode weist einen äußeren Umfang und einen inneren Umfang auf. Der Elektrodenkörper beschreibt dabei eine geschlossene Ringstruktur. Zur Verwendung dieser Elektrode, beispielsweise zur Vaporisierung, zur Resektion oder zur Elektrokoagulation, wird der Toroid mit seiner unteren Hälfte über das zu behandelnde Gewebe eines Patienten bewegt. Durch die toroidale Form weist diese Hochfrequenzelektrode eine große Wirkfläche auf, die andererseits durch die zentrale Öffnung in ihrer Fläche reduziert ist. Durch diese reduzierte aktive Oberfläche ist eine geringere elektrische Energie für die Zündung des Plasmas notwendig als bei bekannten vollflächigen Elektroden. Dadurch lässt sich auch die Zündgeschwindigkeit erhöhen bzw. die Zündzeit verringern. Ein weiterer Vorteil der toroid- oder donutartigen Elektrode ist darin zusehen, dass Bläschen in der Spülflüssigkeit, die durch das Plasma entstehen und die Sicht beeinträchtigen, durch das Loch der Elektrode kontrolliert abgeleitet werden können. Durch dieses Abführen der Bläschen kann die Sicht vor der Optik verbessert werden.

Bevorzugt kann es vorgesehen sein, dass die toroidförmige Elektrode zur Erzeugung eines Plasmas mit einem elektrischen Leiter verbunden ist, über den die Elektrode an einen Hochfrequenzgenerator koppelbar ist. Bei dieser Elektrode ist der elektrische Leiter an einen unteren Bereich bzw. einer unteren Hälfte des Toroids gekoppelt. Dieser untere Bereich ist elektrisch leitend. Der komplementäre obere Bereich bzw. die obere Hälfte der Elektrode kann elektrisch leitend oder isolierend ausgebildet sein. Dadurch wird die elektrische Energie genau dort deponiert, wo das Plasma erzeugt werden soll. Der elektrische Leiter kann dabei beispielsweise durch einen Schaft des Handgerätes, insbesondere des Resektoskopes, zu dem Hochfrequenzgenerator geführt sein. Dabei kann der elektrische Leiter integral sowohl mit der Hochfrequenzelektrode als auch mit dem Handgerät verbunden, oder mit dem Handgerät und/oder der Hochfrequenzelektrode steckerartig koppelbar sein. So ist es denkbar, dass die Hochfrequenzelektrode einen oder zwei Leiter aufweist, über die die Elektrode mit dem Handgerät steckerartig verbindbar ist. Dies gestaltet sich insbesondere für Wartungs- und Reinigungszwecke als besonders vorteilhaft.

Die Erfindung sieht vor, dass die toroidförmige Elektrode zur Erzeugung eines Plasmas zwei elektrische Leiter aufweist, die jeweils einen elektrischen Pol der Elektrode kontaktieren und jeweils mit einem Hochfrequenzgenerator koppelbar sind, wobei die elektrischen Pole der Elektroden durch ein Isolatorelement elektrisch voneinander isoliert sind. Bei dieser bipolaren Elektrode führen die elektrischen Leiter jeweils zu einem Pol an der Elektrode. Dabei ist die Hochfrequenzelektrode in zwei Pole unterteilt. Vorzugsweise ist ein Pol an einer Unterseite und der andere Pol an einer Oberseite der Elektrode positioniert. Zwischen den Polen befindet sich das Isolatorelement. Es ist jedoch auch denkbar, dass die Pole an der Elektrode andersartig aufgeteilt sind. So ist es denkbar, dass ein Pol an der Unterseite der Elektrode wesentlich größer ausgebildet ist als der zweite Pol, der sich nur ringartig auf der Oberseite der Elektrode erstreckt. Durch diese besondere Ausgestaltung der Elektrode lässt sich das für den chirurgischen Einsatz vorgesehen Plasma besonders gut lokalisieren und somit zielgenau einsetzen. Außerdem ist denkbar, dass der andere bzw. der zweite Pol bzw. die Neutralelektrode an einem der beiden Tragarme des Elektrodeninstrumentes angeordnet ist. So ist es denkbar, dass auch die beiden Tragarme bzw. Gabelrohre der Elektrode als Return- und/oder Neutralelektrode dienen können

Darüber hinaus kann es erfindungsgemäß vorgesehen sein, dass die Elektrode über den mindestens einen elektrischen Leiter und/oder über mindestens ein Halteelement an dem Handgerät, insbesondere an einem Elektrodeninstrument, vorzugsweise lösbar, befestigt ist. Neben dem mindestens einen elektrischen Leiter kann somit ein weiteres Element, beispielsweise aus einem Metall oder einem Isolator, die Stabilität der Elektrode an dem Handgerät bzw. dem Elektrodeninstrument sicherstellen. Insbesondere während der Benutzung der Elektrode wirken erhöhte mechanische Zug- und Druckkräfte auf die Elektrode. Das Halteelement und der mindestens eine elektrische Leiter bewirken, dass die Elektrode auch bei erhöhten mechanischen Kräften ihre Position relativ zu dem Handgerät nicht verliert. Auch das Halteelement kann steckerartig ausgebildet sein, sodass eine lösbare Verbindung bzw. eine Koppelverbindung mit dem Handgerät möglich ist.

Die Erfindung sieht vor, dass der mindestens eine, vorzugsweise zwei, elektrische Leiter den Toroid an einer Innenseite kontaktieren. Durch diese Positionierung der Leiter an dem inneren Umfang des Toroids stören die Leiter während der Behandlung nicht. Dadurch, dass die elektrischen Leiter dem inneren Umfang zugeordnet sind, lässt sich der äußere Umfang des Toroids sowie die Unterseite für das Schneiden bzw. Verdampfen des Gewebes einsetzen.

Die Erfindung sieht vor, dass ein Querschnitt parallel zu einer radialen Achse des Toroids elliptisch, oval, kreisartig, dreieckig, rechteckig, vorzugsweise quadratisch, trapezartig, polygonal oder dergleichen ausgebildet ist. Durch diese verschiedenen Querschnitte lassen sich verschiedene Plasmadichten an der Oberfläche des Toroids erzeugen. Insbesondere durch Formen, die spitze Winkel aufweisen, lässt sich eine besonders hohe Plasmadichte erzeugen, die für bestimmte Einsatzzwecke, wie beispielsweise das Schneiden von Gewebe, vorteilhaft sind. Gleichermaßen lasse sich mit großen Flächen Gewebebereiche besonders gut vaporisieren bzw. koagulieren.

Weiter ist es denkbar, dass der äußere Umfang des Toroids elliptisch, oval oder kreisartig ausgebildet ist. Auch durch die Form des Umfangs lässt sich der Einsatz für bestimmte chirurgische Verfahren optimieren. Je nach zu behandelndem Bereich sowie der Art der Behandlung kann es vorteilhaft sein, einen elliptischen, ovalen oder kreisartigen Toroid einzusetzen.

Ein besonderes Ausführungsbeispiel der vorliegenden Erfindung kann es vorsehen, dass um einen äußeren Umfang der Toroidoberfläche ein ringartiger Vorsprung, vorzugsweise eine Kante, ausgebildet ist. Durch diesen Vorsprung wird das Zünden des Plasmas begünstigt bzw. erfolgt das Zünden besonders schnell und zwar mit geringem Energieaufwand. Bedingt durch die höhere elektrische Feldstärke an diesem ringartigen Vorsprung zündet das Plasma schnell, wodurch sich die Elektrode besonders kurzfristig einsetzen lässt. Es ist auch denkbar, dass dieser ringartige Vorsprung nur bereichsweise als Kante ausgebildet ist oder an einer anderen Position auf der Oberfläche des Toroids positioniert ist.

Weiter ist es denkbar, dass ein erster elektrischer Leiter einem unteren Abschnitt des Toroids zugeordnet ist und dieser Abschnitt einen ersten elektrischen Pol bildet und ein zweiter elektrischer Leiter einem oberen Abschnitt des Toroids zugeordnet ist und dieser Abschnitt einen zweiten elektrischen Pol bildet, wobei der untere und der obere Abschnitt flächenmäßig gleichartig oder unterschiedlich ausgebildet sind. Insbesondere ist der untere Abschnitt flächenmäßig größer oder kleiner ist als der obere Abschnitt.

Weiter ist es denkbar, dass ein äußerer, insbesondere mittlerer, Durchmesser des Toroids 2 bis 5 mal, insbesondere 2,5 bis 4, oder 3 mal größer ist als ein innerer, insbesondere mittlerer, Durchmesser des Toroids. Darüber hinaus sind auch noch weitere Verhältnisse der Durchmesser denkbar. So kann es auch vorgesehen sein, dass der Toroid nahezu ringartig ausgebildet ist oder beinahe ganz geschlossen ist. Je nach Einsatzzweck können bestimmte Geometrien besonders vorteilhaft sein.

Der Toroid besteht im Wesentlichen aus Edelstahl, Titan, Platin-Iridium oder Platin-Wolfram. Darüber hinaus ist es denkbar, dass der Toroid auch aus einem anderen elektrisch leitfähigen Material aufgebaut ist. Der elektrische Isolator kann aus einem Kunststoff oder einer Keramik gebildet sein.

Ein Elektrodeninstrument zur Lösung der genannten Aufgabe weist die Merkmale des Anspruchs 10 auf. Demnach ist es vorgesehen, dass das Elektrodeninstrument zur Verwendung in einem chirurgischen Handgerät, insbesondere in einem Resektoskop, einen langgestreckten Schaftabschnitt mit zwei Tragarmen aufweist, durch die hindurch sich mindestens ein Leiter erstreckt, der am distalen Ende des Instruments mit einer Hochfrequenzelektrode gemäß den Ansprüchen 1 bis 9 verbunden bzw. verbindbar ist.

Die Hochfrequenzelektrode ist zwischen den distalen Enden der Tragarme angeordnet. Ein Resektoskop zur Lösung der eingangs genannten Aufgabe weist die Merkmale des Anspruchs 11 auf.

Bevorzugte Ausführungsbeispiele der Erfindung werden nachfolgend anhand der Zeichnung näher beschrieben. In dieser zeigen:
- Fig. 1: eine schematische Darstellung eines chirurgischen Handgeräts, insbesondere eines Resektoskopes,
- Fig. 2: ein Ausführungsbeispiel einer Hochfrequenzelektrode,
- Fig. 3 4: ein weiteres Ausführungsbeispiel einer Hochfrequenzelektrode,
- Fig.: ein weiteres Ausführungsbeispiel einer Hochfrequenzelektrode,
- Fig. 5: ein weiteres Ausführungsbeispiel einer Hochfrequenzelektrode,
- Fig. 6: ein weiteres Ausführungsbeispiel einer Hochfrequenzelektrode, und
- Fig. 7: ein weiteres Ausführungsbeispiel einer Hochfrequenzelektrode.

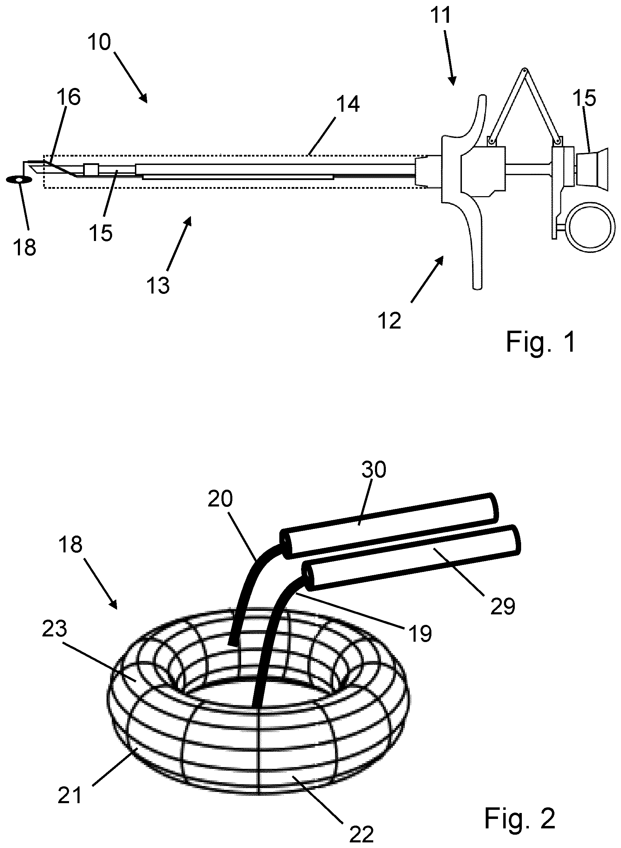

In der Fig. 1 ist beispielhaft für ein chirurgisches Handgerät ein Resektoskop 10 dargestellt. Dieses Resektoskop 10 besteht im Wesentlichen aus einem Transporteur 11, einer Griffeinheit 12 und einem Schaft 13, der zur Behandlung eines Patienten in eine entsprechende Körperöffnung zu führen ist. Bei dem hier dargestellten Ausführungsbeispiel setzt sich der Schaft 13 zusammen aus einem äußeren Schaftrohr 14, einer Optik 15 und einem Elektrodeninstrument 16. Die Optik 15 besteht aus einem langen Rohr, in welchem Linsen oder Glasfasern angeordnet sein können, um durch ein proximal an dem Schaft 13 angeordnetes Okular 17 den Bereich der Behandlung am distalen Ende des Schaftes 13 zu beobachten. Für eine detailliertere Beschreibung eines Resektoskopes wird auf den bekannten Stand der Technik verwiesen.

Das Elektrodeninstrument 16 setzt sich im Wesentlichen zusammen aus einer Hochfrequenzelektrode 18 und mindestens einem elektrischen Leiter 19. Der mindestens eine elektrische Leiter 19 versorgt die Hochfrequenzelektrode 18 zum einen mit einer hochfrequenten Spannung und zum anderen dient der Leiter 19 sowie ggf. ein weiteres Element als Halterung der Elektrode 18 an dem Elektrodeninstrument 16. Der elektrische Leiter 19 führt von dem distalen Ende des Resektoskopes 10 durch den Schaft 14 und ist über weitere Leitungen mit einem nicht dargestellten Hochfrequenzgenerator zur Erzeugung der hochfrequenten elektromagnetischen Energie verbunden. Die beiden Leiter 19, 29 sind durch die beiden Tragarme 29, 30 bzw. durch die Gabelrohre des Elektrodeninstrumentes 16 führbar (Fig. 2)

Mittels der Hochfrequenzelektrode 18 lässt sich beispielsweise Gewebe manipulieren. Dazu kann die Hochfrequenzelektrode 18 entweder als monopolare oder bipolare Elektrode ausgebildet sein. Im Fall einer bipolaren Elektrode ist diese mit zwei elektrischen Leitern 19, 20 verbunden. In dem Ausführungsbeispiel einer monopolaren Elektrode ist die Elektrode 18 nur mit einem elektrischen Leiter 19 verbunden. Eine weitere Neutralelektrode wird an dem Patienten angebracht, oder ist in das Resektoskop integriert (Schaft, Transporteur, Optik; wenn alle Komponenten auf einem elektrischen Potential sind, führt dies aufgrund der großen Fläche zu einer geringen Stromdichte). Durch Beaufschlagung der Hochfrequenzelektrode 18 mit elektrischer Energie wird ein Plasma an der Elektrode 18 erzeugt, mittels welchem durch eine entsprechende Bewegung des Elektrodeninstrumentes 16 Gewebe manipuliert wird.

Die Hochfrequenzelektrode 18 gemäß der vorliegenden Erfindung ist als Toroid 21 ausgebildet. In der Fig. 2 ist beispielhaft ein Toroid 21 mit einem kreisförmigen Querschnitt dargestellt. Dieser Toroid 21 wird über zwei elektrische Leiter 19, 20 des Elektrodeninstrumentes 16 elektrisch mit der Energiequelle verbunden. Alternativ ist es denkbar, dass einer der Leiter 19, 20 als ein Halteelement ausgebildet ist.

Der Toroid 21 bzw. die Hochfrequenzelektrode 18 weist einen unteren Bereich 22 und einen oberen Bereich 23 auf. Diese Bereiche 22, 23 können unterschiedlich groß ausgebildet sein. Bei dem hier dargestellten Ausführungsbeispiel führt der elektrische Leiter 19 zu dem unteren Bereich 22 und der elektrische Leiter 20 zu dem oberen Bereich 23 des Toroids 21. Gleichermaßen ist es denkbar, dass der obere Bereich 23 von dem weiteren Halter fixiert wird. Bei einer bipolaren Elektrode ist zwischen den elektrisch leitenden Bereichen 22 und 23 ein Isolator vorgesehen, um die beiden elektrischen Pole voneinander zu isolieren. Durch diese spezielle Ausführungsform der Hochfrequenzelektrode 18 kann ein besonders großflächiges Plasma am unteren Bereich 22 der Elektrode 18 erzeugt werden, die es erlaubt viel Gewebe in kurzer Zeit abzutragen. Durch die toroidale Form der Hochfrequenzelektrode 18 kann die aktive Oberfläche der Elektrode 18 klein gehalten werden, sodass für die Zündung des Plasmas eine geringe Hitzemenge bzw. wenig elektrische Energie notwendig ist. Außerdem gestaltet sich diese Form besonders vorteilhaft für ein schnelles Zünden des Plasmas.

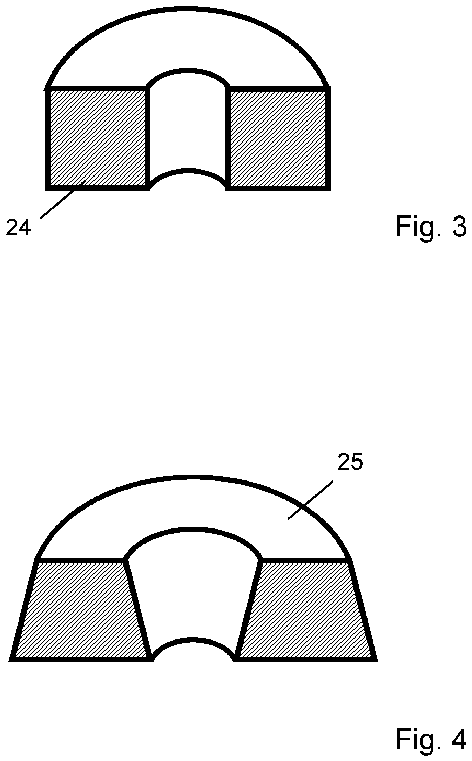

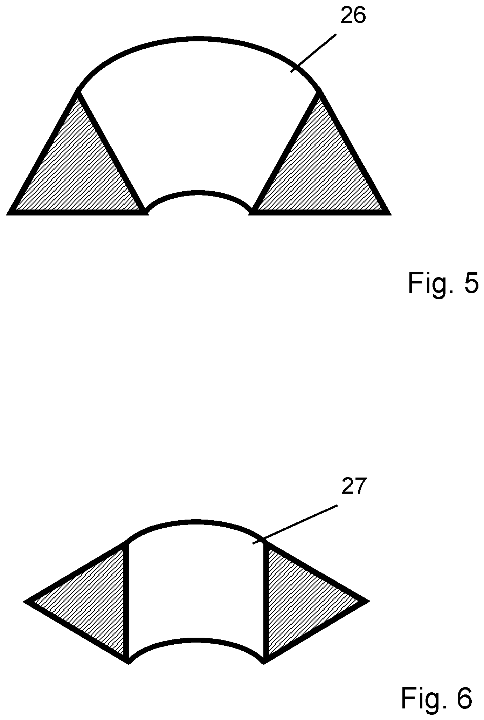

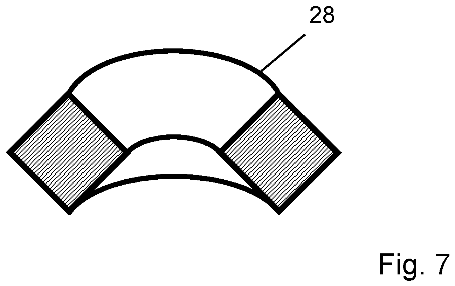

Das Verhältnis zwischen einem äußeren Umfang der "donutförmigen" Hochfrequenzelektrode 18 und einem inneren Umfang kann beliebige Werte annehmen. Je nach Art der Behandlung bzw. des Einsatzes lassen sich verschiedenartig dimensionierte Toroide 21 verwenden. In den Fig. 3 bis 7 sind verschiedene Toroidstrukturen dargestellt. So ist in der Fig. 3 ein Beispiel für eine Hochfrequenzelektrode 18 dargestellt, bei der ein Querschnitt eines Toroids 24 rechteckig bzw. quadratisch ausgebildet ist. Bei dem in der Fig. 4 dargestellten Toroid 25 ist der Querschnitt trapezartig ausgebildet. Das Ausführungsbeispiel in der Fig. 5 zeigt einen Toroid 26 mit einem dreieckigen Querschnitt. Ebenfalls einen dreieckigen Querschnitt zeigt das Ausführungsbeispiel eines Toroids 27 gemäß der Fig. 6. Bei diesem Ausführungsbeispiel ist allerdings das Dreieck derart gekippt, dass eine Spitze nach außen weist. In der Fig. 7 ist ein weiteres Ausführungsbeispiel eines Toroids 28 dargestellt. Bedingt durch die verschiedenen Formen bilden sich aufgrund der verschiedenen elektrischen Feldstärken unterschiedliche Plasmen an der Hochfrequenzelektrode 18 aus. Je nach Anforderung kann somit eine besonders vorteilhaft geformte Elektrode 18 ausgewählt werden. Dabei sei explizit darauf hingewiesen, dass die hier dargestellte Auswahl von Toroidformen nicht abschließend ist. Vielmehr ist es denkbar, dass die Toroide eine nahezu beliebige Form annehmen können.

Ein nicht dargestelltes Ausführungsbeispiel der Erfindung kann es vorsehen, dass an einer Außenseite des Toroids 21 eine Kante bzw. eine ringartige Umrandung angeordnet ist. Diese Kante begünstigt die Ausbildung eines Plasmas, wodurch zum einen die Zündenergie reduzierbar ist und zum anderen die Zündzeit verkürzt werden kann.

Die erfindungsgemäße Hochfrequenzelektrode 18 kann zusammen mit dem Elektrodeninstrument 16 steckerartig mit dem Resektoskop 10 verbindbar sein. Gleichermaßen ist es denkbar, dass die Elektrode 18 steckerartig mit dem Elektrodeninstrument 16 verbindbar ist. Dies ist insbesondere für Wartungs- und Reinigungszwecke besonders vorteilhaft.

Es sei ausdrücklich darauf hingewiesen, dass der Einsatz der Hochfrequenzelektrode 18 nicht auf eine urologische Anwendung beschränkt ist. Vielmehr ist es auch denkbar, dass diese Elektrode 18 für orthopädische oder sonstige chirurgische bzw. medizinische Einsätze Verwendung finden kann.

### Bezugszeichenliste

| | | | |
|---|---|---|---|
| 10 | Resektoskop | 29 | Tragarm |
| 11 | Transporteur | 30 | Tragarm |
| 12 | Griffeinheit | | |
| 13 | Schaft | | |
| 14 | Schaftrohr | | |
| 15 | Optik | | |
| 16 | Elektrodeninstrument | | |
| 17 | Okular | | |
| 18 | Hochfrequenzelektrode | | |
| 19 | Elektrischer Leiter | | |
| 20 | Elektrischer Leiter | | |
| 21 | Toroid | | |
| 22 | Unterer Bereich | | |
| 23 | Oberer Bereich | | |
| 24 | Toroid | | |
| 25 | Toroid | | |
| 26 | Toroid | | |
| 27 | Toroid | | |
| 28 | Toroid | | |

## Patentansprüche

1. Hochfrequenzelektrode (18) zur Verwendung in einem chirurgischen Handgerät, insbesondere in einem Resektoskop (10), wobei die Hochfrequenzelektrode (18) über mindestens einen elektrischen Leiter (19, 20) mit elektrischer Energie versorgbar ist, wobei die Hochfrequenzelektrode (18) die Form eines Toroids (21, 24, 25, 26, 27, 28) aufweist, wobei ein Querschnitt parallel zu einer Radialachse des Toroids (21, 24, 25, 26, 27, 28) elliptisch, oval, kreisförmig, dreieckig, rechteckig, quadratisch, trapezförmig oder polygonal ist, und wobei die elektrischen Leiter (19, 20) den Toroid (21, 24, 25, 26, 27, 28) an einer Innenseite kontaktieren, **dadurch gekennzeichnet, dass** die toroidförmige Elektrode (18) zur Erzeugung eines Plasmas zwei elektrische Leiter (19, 20) aufweist, die jeweils einen elektrischen Pol der Elektrode (18) kontaktieren und mit einem Hochfrequenzgenerator koppelbar sind, wobei die elektrischen Pole der Elektrode (18) durch ein Isolatorelement elektrisch voneinander isoliert sind.

2. Hochfrequenzelektrode (18) nach Anspruch 1, **dadurch gekennzeichnet, dass** wenigstens ein elektrischer Pol, insbesondere eine Neutralelektrode und/oder eine Returnelektrode, der Elektrode (18) durch einen Tragarm (29, 30) bzw. ein Gabelrohr der Elektrode (18) gebildet wird.

3. Hochfrequenzelektrode (18) nach einem der vorangegangenen Ansprüche, **dadurch gekennzeichnet, dass** die Elektrode (18) über den mindestens einen elektrischen Leiter (19, 20) und/oder mindestens an einem Halteelement an dem Handgerät, insbesondere an einem Elektrodeninstrument (16), vorzugsweise lösbar, befestigt ist.

4. Hochfrequenzelektrode (18) nach einem der vorangegangenen Ansprüche, **dadurch gekennzeichnet, dass** die zwei elektrischen Leiter (19, 20) den Toroid (21, 24, 25, 26, 27, 28) an einer Innenseite kontaktieren.

5. Hochfrequenzelektrode (18) nach einem der vorangegangenen Ansprüche, **dadurch gekennzeichnet, dass** ein äußerer Umfang des Toroids (21, 24, 25, 26, 27, 28) elliptisch, oval oder kreisartig ausgebildet ist.

6. Hochfrequenzelektrode (18) nach einem der vorangegangenen Ansprüche, **dadurch gekennzeichnet, dass** um einen äußeren Umfang der Toroidoberfläche ein ringartiger Vorsprung, vorzugsweise eine Kante, ausgebildet ist.

7. Hochfrequenzelektrode (18) nach Anspruch 1, **dadurch gekennzeichnet, dass** ein erster elektrischer Leiter (19) einem unteren Abschnitt des Toroids (21, 24, 25, 26, 27, 28) zugeordnet ist und dieser Abschnitt einen ersten elektrischen Pol bildet und ein zweiter elektrischer Leiter (20) einem oberen Abschnitt des Toroids (21, 24, 25, 26, 27, 28) zugeordnet ist und dieser Abschnitt einen zweiten elektrischen Pol bildet, wobei der untere und der obere Abschnitt flächenmäßig gleichartig oder unterschiedlich ausgebildet sind, insbesondere wobei der untere Abschnitt flächenmäßig größer oder kleiner ist als der obere Abschnitt.

8. Hochfrequenzelektrode (18) nach einem der vorangegangenen Ansprüche, **dadurch gekennzeichnet, dass** ein äußerer, insbesondere mittlerer, Durchmesser des Toroids (21, 24, 25, 26, 27, 28) 2 bis 5 mal, insbesondere 2, 5 bis 4, oder 3 mal größer ist als ein innerer, insbesondere mittlerer, Durchmesser des Toroids (21, 24, 25, 26, 27, 28).

9. Hochfrequenzelektrode (18) nach einem der vorangegangenen Ansprüche, **dadurch gekennzeichnet, dass** der Toroid (21, 24, 25, 26, 27, 28) im Wesentlichen aus Edelstahl, Titan, Platin-Iridium oder Platin-Wolfram besteht und ein elektrischer Isolator aus einer Keramik oder einem Kunststoff.

10. Elektrodeninstrument (16), insbesondere monopolares oder bipolares Elektrodeninstrument (16), zur Verwendung in einem chirurgischen Handgerät, insbesondere in einem Resektoskop (10), wobei das Elektrodeninstrument (16) einen langgestreckten Schaftabschnitt (13) mit zwei Tragarmen (29, 30) aufweist, durch die hindurch sich mindestens ein Leiter (19, 20) erstreckt, der am distalen Ende des Elektrodeninstruments (16) eine mit Hochfrequenzstrom beaufschlagbare Hochfrequenzelektrode (18) gemäß den Ansprüchen 1 bis 9 bildet, die zwischen den distalen Enden der Tragarme (29, 30) angeordnet ist.

11. Resektoskop (10) mit einem Elektrodeninstrument (16) gemäß Anspruch 10.

## Claims

1. A radiofrequency electrode (18) for use in a surgical handheld device, more particularly in a resectoscope (10), wherein the radiofrequency electrode (18) is able to be supplied with electric power by way of at least one electrical conductor (19, 20), wherein the radiofrequency electrode (18) has the shape of a toroid (21, 24, 25, 26, 27, 28), wherein a cross section parallel to a radial axis of the toroid (21, 24, 25, 26, 27, 28) is elliptical, oval, circular, triangular, rectangular, square, trapezoidal or polygonal, and wherein the electrical conductors (19, 20) contact the toroid (21, 24, 25, 26, 27, 28) at an inner side, **characterized in that** the toroidal electrode (18) for generating a plasma comprises two electrical conductors (19, 20), said electrical conductors each contacting an electrical pole of the electrode (18) and being able to be coupled to a radiofrequency generator, wherein the electrical poles of the electrode (18) are electrically insulated from one another by way of an insulator element.

2. The radiofrequency electrode (18) as claimed in claim 1, **characterized in that** at least one electrical pole, more particularly a neutral electrode and/or a return electrode, of the electrode (18) is formed by a support arm (29, 30) or a fork tube of the electrode (18).

3. The radiofrequency electrode (18) as claimed in any one of the preceding claims, **characterized in that** the electrode (18) is fastened, preferably detachably fastened, to the handheld device, in particular to an electrode instrument (16), via the at least one electrical conductor (19, 20) and/or at least on a holding element.

4. The radiofrequency electrode (18) as claimed in any one of the preceding claims, **characterized in that** the two electrical conductors (19, 20) contact the toroid (21, 24, 25, 26, 27, 28) at an inner side.

5. The radiofrequency electrode (18) as claimed in any one of the preceding claims, **characterized in that** an outer circumference of the toroid (21, 24, 25, 26, 27, 28) has an embodiment that is elliptical, oval or circular.

6. The radiofrequency electrode (18) as claimed any one of the preceding claims, **characterized in that** a ring-like protrusion, preferably an edge, is formed around an external circumference of the toroid surface.

7. The radiofrequency electrode (18) as claimed in claim 1, **characterized in that** a first electrical conductor (19) is assigned to a lower section of the toroid (21, 24, 25, 26, 27, 28) and this section forms a first electrical pole and a second electrical conductor (20) is assigned to an upper section of the toroid (21, 24, 25, 26, 27, 28) and this section forms a second electrical pole, wherein the lower and the upper section have the same or a different embodiment in terms of area, in particular wherein the lower section is larger or smaller than the upper section in terms of area.

8. The radiofrequency electrode (18) as claimed in any one of the preceding claims, **characterized in that** an outer diameter, in particular mean outer diameter, of the toroid (21, 24, 25, 26, 27, 28) is 2 to 5 times, more particularly 2.5 to 4, or 3 times larger than an inner diameter, in particular mean inner diameter, of the toroid (21, 24, 25, 26, 27, 28).

9. The radiofrequency electrode (18) as claimed in any one of the preceding claims, **characterized in that** the toroid (21, 24, 25, 26, 27, 28) substantially consists of stainless steel, titanium, platinum iridium or platinum tungsten and an electrical insulator consists of a ceramic or a plastic.

10. An electrode instrument (16), more particularly a monopolar or bipolar electrode instrument (16), for use in a surgical handheld device, more particularly in a resectoscope (10), wherein the electrode instrument (16) comprises an elongate shaft section (13) with two support arms (29, 30), through which at least one conductor (19, 20) extends, the latter forming a radiofrequency electrode (18) as claimed in claims 1 to 9, which is able to be impinged with a radiofrequency current, at the distal end of the electrode instrument (16), said radiofrequency electrode being arranged between the distal ends of the support arms (29, 30).

11. A resectoscope (10) comprising an electrode instrument (16) as claimed in claim 10.

## Revendications

1. Électrode haute fréquence (18) destinée à être utilisée dans un appareil chirurgical portable, en particulier dans un résectoscope (10), l'électrode haute fréquence (18) pouvant être alimentée en énergie électrique par l'intermédiaire d'au moins un conducteur électrique (19, 24), l'électrode haute fréquence (18) présentant la forme d'un tore (21, 20, 25, 26, 27, 28), une section transversale parallèle à un axe radial du tore (21, 24, 25, 26, 27, 28) étant elliptique, ovale, circulaire, triangulaire, rectangulaire, carrée, trapézoïdale ou polygonale, et les conducteurs électriques (19, 20) étant en contact avec le tore (21, 24, 25, 26, 27, 28) sur une face intérieure, **caractérisée en ce que** l'électrode toroïdale (18) comporte deux conducteurs électriques (19, 20) destinés à la production d'un plasma et qui sont respectivement en contact avec un pôle électrique de l'électrode (18) et peuvent être couplés à un générateur haute fréquence, les pôles électriques de l'électrode (18) étant isolés électriquement l'un de l'autre par un élément isolant.

2. Électrode haute fréquence (18) selon la revendication 1, **caractérisée en ce qu'**au moins un pôle électrique, en particulier une électrode neutre et/ou une électrode de retour, de l'électrode (18) est formé par un bras de support (29, 30) ou un tube en fourche de l'électrode (18).

3. Électrode haute fréquence (18) selon l'une des revendications précédentes, **caractérisée en ce que** l'électrode (18) est fixée, de préférence de manière amovible, à l'appareil portable, en particulier à un instrument à électrode (16), par l'intermédiaire dudit au moins un conducteur électrique (19, 20) et/ou au moins à un élément de retenue.

4. Électrode haute fréquence (18) selon l'une des revendications précédentes, **caractérisée en ce que** les deux conducteurs électriques (19, 20) sont en contact avec le tore (21, 24, 25, 26, 27, 28) sur une face intérieure.

5. Électrode haute fréquence (18) selon l'une des revendications précédentes, **caractérisée en ce qu'**une périphérie extérieure du tore (21, 24, 25, 26, 27, 28) est réalisée de manière elliptique, ovale ou circulaire.

6. Électrode haute fréquence (18) selon l'une des revendications précédentes, **caractérisée en ce qu'**une saillie annulaire, de préférence un bord, est formée autour d'une périphérie extérieure de la surface toroïdale.

7. Électrode haute fréquence (18) selon la revendication 1, **caractérisée en ce qu'**un premier conducteur électrique (19) est associé à une partie inférieure du tore (21, 24, 25, 26, 27, 28) et **en ce que** cette partie forme un premier pôle électrique, et **en ce qu'**un deuxième conducteur électrique (20) est associé à une partie supérieure du tore (21, 24, 25, 26, 27, 28) et cette partie forme un deuxième pôle électrique, la partie intérieure et la partie supérieure étant réalisées de manière à présenter des surfaces identiques ou différentes, la partie intérieure présentant en particulier une surface plus grande ou plus petite que la partie supérieure.

8. Électrode haute fréquence (18) selon l'une des revendications précédentes, **caractérisée en ce qu'**un diamètre extérieur, en particulier moyen, du tore (21, 24, 25, 26, 27, 28) est 2 à 5 fois, en particulier 2, 5 à 4 fois ou 3 fois supérieur à un diamètre intérieur, en particulier moyen, du tore (21, 24, 25, 26, 27, 28).

9. Électrode haute fréquence (18) selon l'une des revendications précédentes, **caractérisée en ce que** le tore (21, 24, 25, 26, 27, 28) est constitué pour l'essentiel d'acier inoxydable, de titane, de platine-iridium ou de platine-tungstène et d'un isolant électrique en céramique ou en matière plastique.

10. Instrument à électrode (16), en particulier instrument à électrode monopolaire ou bipolaire (16), destiné à être utilisé dans un appareil chirurgical portable, en particulier dans un résectoscope (10), **caractérisé en ce que** l'instrument à électrode (16) présente une partie tige allongée (13) pourvue de deux bras de support (29, 30) traversés par au moins un conducteur (19, 20), qui forme à l'extrémité distale de l'instrument à électrode (16) une électrode haute fréquence (18) pouvant être alimentée en courant haute fréquence selon les revendications 1 à 9, qui est disposée entre les extrémités distales des bras de support (29, 30).

11. Résectoscope (10) comprenant un instrument à électrode (16) selon la revendication 10.
